# FASCICULE DE BREVET EUROPEEN

(11) **EP 4 127 664 B1**
(45) Date de publication et mention de la délivrance du brevet: **10.12.2025**
(21) Numéro de dépôt: 21716395.5
(22) Date de dépôt: 01.04.2021
(51) Int. Cl.: G01N 21/35, C12Q 1/04, G01N 21/03

(54) **PROCÉDÉ DE CARACTÉRISATION DE MICROORGANISMES PAR IMAGERIE EN TRANSMISSION**
VERFAHREN ZUR CHARAKTERISIERUNG VON MIKROORGANISMEN MITTELS TRANSMISSIONSBILDGEBUNG
METHOD FOR CHARACTERISING MICRO-ORGANISMS USING TRANSMISSION IMAGING

(30) Priorité: 03.04.2020 FR 2003377
(43) Date de publication de la demande: 08.02.2023
(73) Titulaire: Commissariat à l'Energie Atomique et aux Energies Alternatives, 75015 Paris (FR)
(72) Inventeur: MARCOUX, Pierre, 38054 Grenoble Cedex 09 (FR); BIARDEAU, Victor, 38054 Grenoble Cedex 09 (FR); DUPOY, Mathieu, 38054 Grenoble Cedex 09 (FR); GAILLARD, Frédéric-Xavier, 38054 Grenoble Cedex 09 (FR); LE GALUDEC, Joel, 38054 Grenoble Cedex 09 (FR)
(74) Mandataire: INNOV-GROUP
(86) Numéro de dépôt international: PCT/EP2021/058664
(87) Numéro de publication internationale: WO 2021/198443

(56) Documents cités:
- WO-A1-2015/107228
- WO-A2-2006/112713
- US-A1- 2010 190 204
- US-A1- 2017 167 973
- "Anopore inorganic aluminum oxide membrane filters - smallest pore sizes possible of any membrane filter", INTERNET CITATION, 1 January 2005 (2005-01-01), XP002987479, Retrieved from the Internet <URL:http://secure.2spi.com/catalog/spec_prep/filter2.shtml> [retrieved on 20050726]

## Description

### DOMAINE TECHNIQUE

Le domaine technique de l'invention est la caractérisation de microorganismes par une image acquise selon une configuration en transmission, dans l'infra-rouge.

### ART ANTERIEUR

Il existe de nombreuses méthodes permettant de caractériser des colonies de microorganismes, cultivées dans des milieux nutritifs liquides (bouillons) ou sur des géloses. Une première méthode consiste à évaluer différents paramètres, visuels ou olfactifs, de colonies. Ces paramètres peuvent être la forme des colonies, l'état de surface, la couleur ou l'odeur. Cette méthode peut être appliquée par un opérateur expérimenté, de façon individuelle, c'est-à-dire colonie par colonie.

Certaines méthodes, automatisées et plus précises, sont destructives. Elles nécessitent de prélever les colonies de leur milieu nutritif. Il s'agit par exemple de techniques basées sur la spectrométrie de masse, de type MALDI TOF (Matrix-Assisted Laser Desorption lonization -Time of Flight - signifiant désorption-ionisation par laser assistée par matrice - temps de vol). Il s'agit d'une méthode performante. Cependant, elle nécessite un équipement complexe, et doit être effectuée individuellement, pour chaque colonie. De plus, il s'agit d'une technique destructive. Une autre technique, potentiellement destructive, utilisée est la spectrométrie Raman. Il s'agit également d'une technique individuelle, mise en œuvre avec un équipement sophistiqué, nécessitant une analyse colonie par colonie.

Des techniques d'imagerie multispectrales peuvent être utilisées. Il s'agit d'illuminer des colonies selon différentes longueurs d'onde, dans le domaine spectral visible ou ultraviolet ou infra-rouge. Dans le visible, par exemple, on peut caractériser des colonies de bactéries à partir de figures de diffraction.

Lorsque le milieu nutritif est suffisamment transparent, ce dernier peut être disposé entre une source de lumière et un capteur d'image, selon une configuration en transmission. Le capteur d'image peut être utilisé sans lentille, c'est-à-dire en l'absence d'optique de formation d'image entre le milieu nutritif et le capteur d'image. Une telle configuration permet l'obtention de figures de diffraction, dans une ou plusieurs longueurs d'onde. Ces dernières constituent des signatures, permettant l'identification des espèces de microorganismes présentes dans les colonies. Des exemples d'application sont par exemple décrits dans US20160103064 ou dans US7465560.

Lorsque le milieu nutritif est opaque, la configuration en transmission n'est pas envisageable. Des applications en rétrodiffusion ont été décrites dans US20190339199 ou dans US20190323959. L'objectif est d'obtenir une image représentative d'un rayonnement rétrodiffusé par une colonie bactérienne. Compte tenu des contraintes de géométrie en rétrodiffusion, cette méthode est principalement limitée à une caractérisation individuelle de colonies.

WO2006112713 décrit une analyse d'un échantillon, comportant des microorganismes par imagerie de fluorescence. Les microorganismes sont supportés par un support microporeux.

US20100190204 décrit une analyse d'un échantillon, comportant des microorganismes, par colorimétrie.

WO2015107228 décrit un support poreux permettant de retenir des microorganismes tout en permettant leur nutrition.

US2017167973 décrit une analyse de microorganismes, prélevés d'un milieu de culture, par imagerie spectrale infra-rouge.

Dans le domaine de l'infra-rouge, l'application de l'imagerie en transmission est compliquée par le fait que les milieux nutritifs, sur lesquels les microorganismes se développent, sont absorbants. Cela est notamment dû à la forte fraction massique d'eau, pouvant être supérieure à 80%.

Les inventeurs ont développé un procédé optique de caractérisation de microorganismes, dans le domaine de l'infra-rouge, selon une configuration de transmission : les microorganismes caractérisés sont disposés entre une source de lumière et un capteur d'image.

### EXPOSE DE L'INVENTION

Un objet de l'invention est un procédé de caractérisation de microorganismes, comportant :
a) dépôt de microorganismes sur un support poreux, le support poreux comportant une première face et une deuxième face, et des pores s'étendant de la première face jusqu'à la deuxième face, les microorganismes étant retenus sur la première face ;
b) disposition du support poreux à la surface d'un milieu nutritif, contenu dans une enceinte, le support poreux étant disposé de telle sorte que la deuxième face est disposée au contact du milieu nutritif, de façon que le milieu nutritif diffuse de la deuxième face vers la première face, à travers les pores;
c) déplacement du support poreux par rapport à l'enceinte ;
d) positionnement du support poreux entre une source de lumière infra-rouge et un capteur d'image, la source de lumière étant configurée pour émettre une onde lumineuse incidente dans une longueur d'onde d'émission, le support poreux transmettant tout ou partie de l'onde lumineuse incidente à la longueur d'onde d'émission ;
e) illumination des microorganismes, disposés sur le support poreux, par la source de lumière et acquisition d'une image par le capteur d'image, à la longueur d'onde d'émission, l'image permettant une observation d'au moins une colonie de microorganismes ;
f) caractérisation de la colonie de microorganismes à partir de l'image acquise lors de l'étape e).

Le milieu nutritif comporte des nutriments propices au développement de colonies de microorganismes. Il peut comporter un agent bactéricide ou bactériostatique. Il peut également comporter un marqueur isotopique, ou un marqueur chromogène, ou une molécule présentant une liaison absorbant la lumière infra-rouge dans une longueur d'onde correspondant à la longueur d'onde d'émission.

Selon l'invention, la caractérisation est :
- une identification de l'espèce des microorganismes formant la colonie ;
- ou une détermination de la capacité des microorganismes à se développer dans le milieu nutritif.

Selon l'invention :
- l'étape e) est répétée en illuminant les microorganismes successivement à différentes longueurs d'onde d'émission, de façon à obtenir autant d'images que de longueurs d'onde d'émission ;
- lors de l'étape f), la caractérisation est effectuée à partir des images acquises lors de l'étape e).

De préférence, le support poreux transmet au moins 1% de la lumière dans la longueur d'onde d'émission, ou dans chaque longueur d'onde d'émission.

L'étape f) peut comporter une extraction d'une vignette à partir de l'image acquise lors de l'étape e), la vignette étant une région d'intérêt de l'image acquise correspondant à la colonie de microorganismes. Lors de l'étape f), la vignette, ou chaque vignette, peut former une donnée d'entrée d'un algorithme à apprentissage supervisé, de façon à caractériser les microorganismes formant une colonie.

Selon un mode de réalisation, l'étape a) comporte un ensemencement du support poreux.

Selon un mode de réalisation, l'étape a) comporte un écoulement d'un fluide, susceptible de comporter des microorganismes, à travers le support poreux, de la première face vers la deuxième face, le support agissant en tant que filtre, de façon à retenir les microorganismes sur la première face, tout en autorisant un écoulement du fluide à travers les pores. Le fluide peut être un liquide ou un gaz.

Selon un mode de réalisation, lors de l'étape b), lequel le milieu nutritif comporte :
- des molécules marquées par un isotope ;
- ou est un milieu chromogène ;
- ou comporte des molécules présentant une liaison chimique absorbant la lumière à la longueur d'onde d'émission ;
de telle sorte que l'image formée lors de l'étape e), ou chaque image formée lors de l'étape e) est représentative d'une activité métabolique des microorganismes au contact avec le milieu nutritif.

Selon un mode de réalisation, lors de l'étape b), le milieu nutritif comporte un agent bactéricide ou un agent bactériostatique, par exemple un antibiotique, de telle sorte que l'image formée lors de l'étape e), ou chaque image formée lors de l'étape e) est représentative d'une activité métabolique des microorganismes lorsqu'ils sont en contact avec le milieu nutritif.

Selon un mode de réalisation, suite à l'étape a) et préalablement à l'étape b), le procédé comporte une acquisition d'une image initiale du support poreux, de telle sorte que l'activité métabolique est déterminée en fonction d'une comparaison entre l'image initiale et au moins une image acquise lors de l'étape e).

L'épaisseur du support poreux, entre la première face et la deuxième face, est de préférence inférieure à 1 mm ou à 500 µm. Le diamètre ou la plus grande diagonale de chaque pore peut être compris entre 5 nm et 5 µm, et de préférence entre 20 nm et 500 nm. Le support poreux peut être obtenu à partir d'un matériau choisi parmi : l'alumine, le silicium, le germanium, le sulfure de zinc, le nitrure de silicium, le séléniure de zinc, un verre de chalcogénure, le fluorure de calcium, le bromure de potassium.

L'invention sera mieux comprise à la lecture de l'exposé des exemples de réalisation présentés, dans la suite de la description, en lien avec les figures listées ci-dessous.

### FIGURES

La figure 1A montre un exemple de support poreux convenant à la mise en œuvre de l'invention.
La figure 1B schématise une mise en contact du support poreux, représenté sur la figure 1A, à la surface d'un milieu nutritif.
La figure 1C montre un montage permettant d'effectuer une image de colonies de microorganismes disposées sur le support poreux, selon une configuration en transmission.
La figure 2A trace d'une part un spectre de l'absorbance d'un support poreux en alumine, et d'autre part les spectres d'absorbance de différents microorganismes.
La figure 2B est une observation, au microscope électronique, d'une partie d'une face d'un support poreux. Cette figure illustre les pores formés dans le support poreux.
La figure 2C est une observation, au microscope électronique, d'une coupe d'un support poreux. Cette figure illustre que les pores s'étendent entre les deux faces du support poreux.
La figure 3 schématise les principales étapes d'un procédé de mise en œuvre de l'invention, ainsi que de variantes du procédé.
La figure 4A est une photographie d'un milieu nutritif, sur une partie duquel on a déposé un support poreux. Cette photographie montre la capacité du support poreux à permettre un développement de colonies de microorganismes.
Les figures 4B et 4C illustrent le développement de microorganismes respectivement sur une gélose et sur un support poreux déposé sur une gélose.
Les figures 5A, 5B, 5C,5D et 5E sont des images de colonies de microorganismes obtenues en mettant en œuvre un montage tel que décrit en lien avec la figure 1C, dans plusieurs longueurs d'onde. Les figures 5A, 5B, 5C, 5D et 5E correspondent respectivement à différentes espèces bactériennes.
Les figures 6A à 6F illustrent un mode de réalisation, dans lequel le support poreux agit en tant que filtre, pour collecter des microorganismes, ainsi qu'en tant que support des microorganismes, durant leur développement, ainsi qu'en tant que support des microorganismes durant l'acquisition d'images.
Les figures 7A à 7D illustrent un mode de réalisation dans lequel les microorganismes sont successivement mis en contact d'un milieu nutritif initial, puis d'un deuxième milieu nutritif. Selon ce mode de réalisation, le deuxième milieu nutritif comporte des molécules marquées par un marqueur, par exemple un marqueur isotopique, ou un milieu enzymatique chromogène.
La figure 7E représente respectivement des colonies au contact d'un milieu chromogène.

### EXPOSE DE MODES DE REALISATION PARTICULIERS

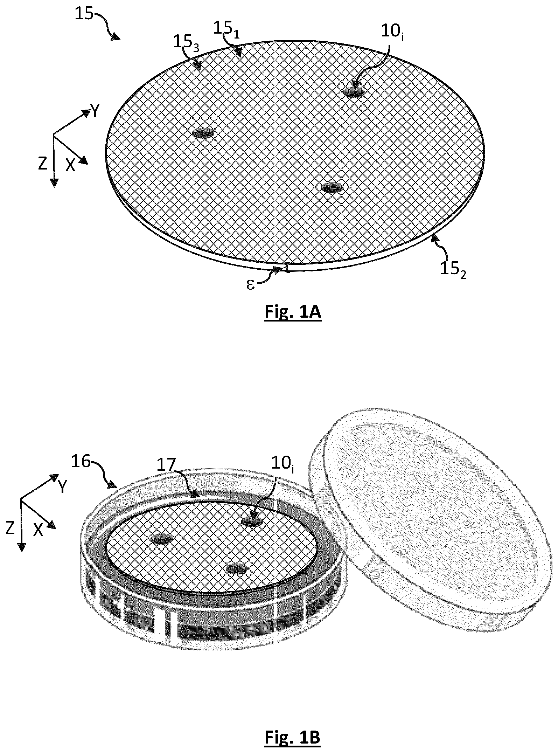

La figure 1A schématise un support poreux 15 mis en œuvre dans l'invention. Le support poreux 15 s'étend entre une première face 15₁ et une deuxième face 15₂, selon une épaisseur ε. De préférence, les première et deuxième face sont planes, parallèles l'une à l'autre. Elles forment alors deux faces opposées du support.

Le diamètre, ou la plus grande diagonale, de chaque face peut par exemple être compris entre 1 cm et 10 cm, voire davantage. L'épaisseur ε du support poreux 15 est de préférence inférieure à 500 µm ou à 1 mm ou à 2 mm. Elle est de préférence comprise entre 10 µm et 1 mm, ou entre 10 µm et 500 µm. En fonction de l'épaisseur et du matériau formant le support, le support poreux 15 est souple ou rigide.

Le support poreux 15 comporte des pores 15₃, s'étendant de la première face 15₁ jusqu'à la deuxième face 15₂.

Une des fonctions du support poreux 15 est de former un support à des microorganismes, et généralement des colonies de microorganismes 10ᵢ, disposés sur une des faces. Dans l'exemple représenté, les microorganismes 10ᵢ sont disposés sur la première face 15₁ du support 15. Le terme microorganismes inclut les bactéries, les archées, les champignons microscopiques tels que les levures ou les champignons filamenteux, les microalgues, les protozoaires et les parasites.

Le diamètre, ou la plus grande diagonale, de chaque pore 15₃, est de préférence inférieur à 5 µm, voire 1 µm, voire 500 nm, voire 200 nm, et supérieur à 1nm, voire 5 nm, voire 20 nm, voire 50 nm. Le diamètre ou la plus grande diagonale est défini de manière à permettre un maintien des microorganismes sur la première face 15₁ du support.

La figure 1B représente le support poreux 15 déposé à la surface d'un milieu nutritif 17. Le milieu nutritif 17 est retenu dans une enceinte 16. L'enceinte 16 est par exemple une boite de Petri. Le milieu nutritif 17 comporte des nutriments propices au développement de colonies de microorganismes. Il peut par exemple s'agir d'une gélose, ou d'un bouillon liquide, ces types de milieu nutritif étant connu de l'homme du métier. Le support poreux 15 est déposé sur une surface du milieu nutritif. La deuxième face 15₂ est au contact avec le milieu nutritif 17. Le milieu nutritif diffuse à travers les pores 15₃ jusqu'à la première face 15₁. De ce fait, les microorganismes disposés sur la première face 15₁ sont alimentés par le milieu nutritif 17 et peuvent se développer.

Les pores 15₃ permettent une diffusion du milieu nutritif 17 entre les deux faces du support 15, tout en retenant les microorganismes sur une face du support. La dimension des pores 15₃ est adaptée de façon à être :
- suffisamment large pour permettre une diffusion du milieu 17 à travers les pores;
- suffisamment étroite pour bloquer un passage des microorganismes 10ᵢ.

Ainsi, les microorganismes sont retenus sur la première face 15₁, tout en se développant grâce à l'apport de nutriments à travers les pores 15₃.

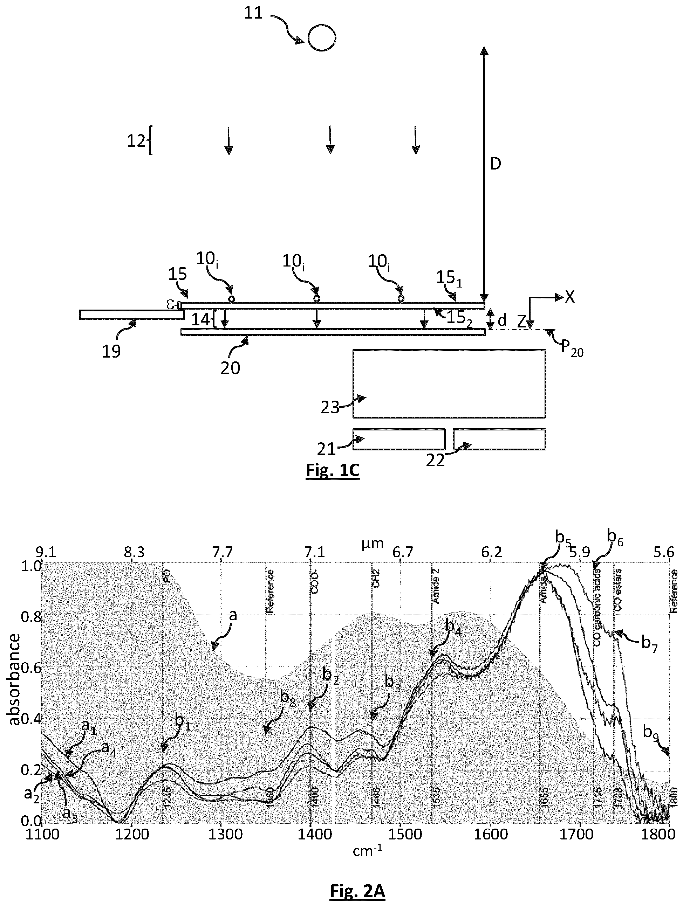

La figure 1C montre une utilisation du support 15 en tant que support d'analyse de colonies de microorganismes 10ᵢ. Le support 15 est retiré du milieu nutritif 17 et est éloigné de l'enceinte 16, de façon être disposé face à une source de lumière 11 et face à un détecteur 20. Dans l'exemple représenté sur la figure 1C, le support est disposé entre la source de lumière 11 et un capteur d'image 20.

La source de lumière 11 peut être une diode électroluminescente ou une source laser. La source de lumière 11 émet une onde lumineuse incidente 12, dans une longueur d'onde d'émission λ. La longueur d'onde d'émission λ se situe dans le domaine spectral de l'infra-rouge. Il peut s'agir de l'infrarouge court, usuellement désigné par l'acronyme SWIR (Short Wavelenght Infrared), s'étendant entre 1 et 3 µm, ou du moyen infrarouge, usuellement désigné par l'acronyme MWIR (Medium Wavelenght Infrared), s'étendant entre 3 et 6 µm, ou encore de l'infrarouge long, usuellement désigné par l'acronyme LWIR (Long Wavelenght Infrared), s'étendant entre 6 et 20 µm. Ainsi, d'une façon générale, l'onde lumineuse incidente 12 est émise selon une longueur d'onde λ s'étendant entre 1 µm et 20 µm ce qui correspond à un nombre d'onde compris entre 500 cm⁻¹ et 10000 cm⁻¹, ou entre 3 µm et 20 µm. La plage spectrale préférée se situe entre 3 µm et 11 µm, qui correspond au moyen infra-rouge, et d'une partie de l'infra-rouge long. En effet, cette plage spectrale correspond au domaine des longueurs d'onde d'absorption de liaisons covalentes usuellement rencontrées dans les molécules organiques.

La source de lumière 11 est de préférence une source laser. Il peut notamment s'agir d'une source laser accordable en longueur d'onde, par exemple un laser QCL, acronyme de Quantum Cascade Laser, signifiant laser à cascade quantique, en particulier un laser à cavité externe. La largeur de la bande spectrale d'émission de la source de lumière de préférence inférieure à 10 cm⁻¹ nm, voire à 5 cm⁻¹, voire à 1 cm⁻¹. La distance D entre la source de lumière et le support peut être de quelques cm.

La source de lumière 11 peut comporter plusieurs sources laser élémentaires QCL, émettant respectivement dans différentes bandes spectrales. La source de lumière 11 peut également être une source polychromatique, de type corps noir, pouvant être associée à différents filtres passe-bande, définissant la longueur d'onde d'émission λ. Ainsi, la source de lumière 11 peut être polychromatique et accordable en longueur d'onde.

Le support 15 est disposé entre la source de lumière 11 et le capteur d'image 20. Ce dernier s'étend de préférence parallèlement, ou sensiblement parallèlement à une face du support 15. Le terme sensiblement parallèlement signifie que les deux éléments peuvent ne pas être rigoureusement parallèles, une tolérance angulaire de quelques degrés, inférieure à 20° ou 10° étant admise. Le support 15 peut être disposé sur un élément de maintien 19, configuré pour maintenir le support, entre la source de lumière 11 et le capteur d'image 20.

Sous l'effet de l'illumination par l'onde lumineuse incidente 12, se propageant selon l'axe de propagation Z jusqu'au support 15, ce dernier transmet une onde lumineuse 14, dite onde lumineuse transmise. L'onde lumineuse transmise 14 se propage, selon l'axe Z, jusqu'au capteur d'image 20. Le support 15 et les microorganismes 10ᵢ peuvent absorber une partie de l'onde lumineuse incidente 12. Aussi, l'onde lumineuse transmise 14 correspond à une partie de l'onde lumineuse incidente 12 non absorbée par le support 15 et par les colonies de microorganismes 10ᵢ.

Le capteur d'image 20 est apte à former une image de l'onde lumineuse transmise 14 selon un plan de détection P₂₀. Dans cet exemple, le capteur d'image est formé par une matrice de pixels. Chaque pixel est un détecteur infra-rouge élémentaire. Chaque pixel peut par exemple être un bolomètre, chaque bolomètre de la matrice présentant une bande spectrale de détection comprise entre 5 µm et 20 µm. La matrice de bolomètres peut par exemple comporter 80 x 80 bolomètres. Selon des variantes, le capteur d'image peut comporter une matrice de pyrodétecteurs, ou de photodiodes, dont la bande spectrale de détection se situe dans l'infra-rouge, et de préférence dans l'infra-rouge moyen. Ainsi, chaque pixel du capteur d'image 20 peut être un détecteur élémentaire infra-rouge :
- de type thermique, par exemple thermorésistif ou thermocapacitif ou thermoélectrique ;
- ou de type photodétecteur quantique, par exemple de type photocapacité, photoconducteur, photodiode, QWIP (Quantum well infrared photodetector, signifiant photodétecteur infrarouge à puits quantique).

Les matériaux formant chaque pixel peuvent être choisis, à titre non limitatif, parmi le PZT (Titano zarconate de plomb), InSb (Antimoniure d'indium), HgCdTe (Tellurure de mercure-cadmium), PbS (Sulfure de plomb), PbSe (Selenure de plomb).

La distance d entre le capteur d'image 20 et le support 15 est de préférence inférieure à 5 mm. Plus elle est réduite, meilleure est la résolution spatiale de l'image acquise par le capteur d'image. Aussi, il est avantageux que la distance d soit inférieure à 1 mm, voire inférieure à 500 µm.

Le champ d'observation du capteur d'image 20 est défini par la taille de la surface sensible de ce dernier. Le champ d'observation peut être supérieur à 1 mm², voire supérieur à 5 mm² ou 10 mm². De ce fait, l'acquisition d'une seule image permet d'obtenir simultanément une intensité de l'onde lumineuse 14 transmise par une surface du support de plusieurs mm², typiquement au moins 5 ou 10 mm². Une telle configuration permet une observation simultanée de plusieurs colonies de microorganismes espacées les unes des autres.

L'élément de maintien 19 peut être mobile, en particulier parallèlement au plan de détection P₂₀, de façon à permettre un balayage de tout ou partie de la surface du support 15. Il peut en particulier s'agir d'une platine de translation motorisée. L'élément de maintien 19 peut également permettre une rotation du support 15 parallèlement au plan de détection.

Une unité de traitement, prenant par exemple la forme d'un microprocesseur 21, est configurée pour effectuer, à partir des images acquises par le capteur d'image 20, des opérations d'interprétation d'images décrites par la suite. Le microprocesseur 21 est relié à une mémoire 22, comportant des instructions relatives aux traitements d'images à effectuer. Il peut être relié à un écran 23.

Un élément important de l'invention réside dans le fait que le support poreux 15 permet une transmission d'une partie de l'onde lumineuse incidente 12 vers le capteur d'image 20. De préférence, la transmittance est supérieure à 0.01 (1%) ou supérieure à 0.1 (10%). Par transmittance, on entend un pourcentage de l'intensité lumineuse transmise par le matériau formant le support poreux 15. Il est avantageux que la transmittance soit la plus élevée possible.

Le matériau composant le support poreux 15 peut être constitué de ou comporter un matériau ayant de bonnes propriétés de transmission dans l'infra-rouge. Il peut par exemple s'agir d'un des matériaux suivants : alumine (oxyde d'aluminium), silicium (Si), germanium (Ge), sulfure de zinc (ZnS), séléniure de zinc (ZnSe), des verres de chalcogénures (chalcogenide glasses) à base de soufre, ou de sélénium ou de tellure, nitrure de silicium (Si₃N₄), du fluorure de calcium (CaF₂) ou du bromure de potassium (KBr).

Le recours au support poreux 15 permet d'utiliser un détecteur 20 selon un mode de transmission, le support 15 étant interposé entre la source de lumière et le détecteur 20. Cela permet une caractérisation des colonies de microorganismes, prises individuellement, à partir de l'onde lumineuse 14 transmise par le support 15 et collectée par le détecteur 20. Lorsque le détecteur 20 est un capteur d'image, sensible à l'infra-rouge, chaque image acquise I_{λ} par le capteur d'image permet une caractérisation des microorganismes. Plus précisément, chaque image acquise I_{λ} est représentative d'une absorption de l'onde lumineuse incidente 12 par les microorganismes, et par le support poreux 15, à la longueur d'onde λ.

On comprend que le support poreux 15 présente une double fonction :
- support des microorganismes lors de leur développement, en étant mis en contact avec un milieu nutritif ;
- support des microorganismes lors d'une analyse par un procédé optique, en particulier un procédé d'imagerie infra-rouge en transmission.

Le recours au support poreux 15 permet de surmonter l'impossibilité de former une image en transmission de microorganismes se développant sur des milieux nutritifs opaques dans le domaine spectral infra-rouge. L'utilisation du support poreux 15 permet de déplacer les microorganismes entre des conditions de culture, telles que représentées sur la figure 1B, et un dispositif d'analyse, tel que schématisé sur la figure 1C. Un avantage notable est que le développement des microorganismes et leur analyse sont effectués sur un même support 15, sans nécessiter un transfert. La morphologie et la structure des colonies de microorganismes sont ainsi conservées entre les conditions de culture et les conditions d'analyse.

Par ailleurs, le procédé d'analyse par image en transmission n'est pas destructif. Il permet, suite à l'acquisition d'une image, de placer à nouveau le support sur un milieu nutritif. Ce dernier peut être identique ou différent d'un milieu nutritif initial, utilisé préalablement à l'acquisition de l'image. On peut ainsi déplacer les microorganismes entre un milieu nutritif initial et un autre milieu nutritif, différent du milieu nutritif initial. Cela permet d'évaluer la capacité des microorganismes à se développer dans l'autre milieu nutritif, comme décrit par la suite.

Comme précédemment indiqué, le procédé permet d'adresser un champ d'observation large. En effet, il est possible de disposer le support à faible distance du capteur d'image 20, par exemple à une distance d inférieure à 1 cm, voire au contact du capteur d'image. Il en résulte un champ d'observation important, dépendant essentiellement de la taille du capteur d'image. Le fait de disposer le capteur d'image à une distance d suffisamment faible du support permet d'éviter le recours à un système optique de formation d'image entre le capteur d'image 20 et le support 15.

Outre de bonnes propriétés de transmittance dans l'infra-rouge, le matériau formant le support 15 est de préférence non cytotoxique à l'égard des microorganismes caractérisés. Il est de préférence stérilisable, en étant compatible avec un des procédés de stérilisations usuels : stérilisation par exposition à des rayonnements ionisants (rayonnement gamma ou X par exemple) ou non ionisants (micro-ondes ou rayonnement ultraviolet), stérilisation chimique (agents oxydants forts) ou thermique (chaleur humide, chaleur sèche, autoclave).

La formation de pores 15₃ dans le matériau formant le support peut résulter de l'application d'un procédé chimique, par exemple une oxydation électrochimique, ou d'un procédé physique, par exemple une exposition à un faisceau d'électrons ou d'ions lourds. Le procédé de formation de pores doit permettre l'obtention de pores 15₃ transverses, (i-e s'étendant de la première face 15₁ à la deuxième face 15₂) et de préférence de taille contrôlée.

La figure 2A montre un exemple d'un spectre de l'absorbance de l'alumine (courbe a), pour un spectre en longueur d'onde s'étendant entre 5.6 µm et 8 µm (nombre d'onde compris entre 1200 cm⁻¹ et 1800 cm⁻¹). L'absorbance est égale à 1 moins la transmittance. Sur la figure 2A, l'axe des ordonnées correspond à l'absorbance (comprise entre 0 et 1). Les axes des abscisses correspondent au nombre d'onde (axe du bas - unité cm⁻¹) et aux longueurs d'onde (axe du haut - unité micromètre µm).

On a également représenté des spectres d'absorbance de différents microorganismes : *Candida albicans (courbe a1) , Escherichia coli (courbe a2), Listeria Inoccua* (courbe *a3*), *Enterobacter cloacae* (courbe *a4)*. Les pics d'absorption de différentes liaisons chimiques ont également été matérialisés par des pointillés. Ces pics sont répertoriés dans le tableau 1.

**Tableau 1**

| Référence | Nombre d'onde (cm⁻¹) | Longueur d'onde (µm) | Liaison chimique |
|---|---|---|---|
| b₁ | 1235 | 8.1 | Phosphodiester |
| b₂ | 1400 | 7.14 | Carboxylate |
| b₃ | 1468 | 6.83 | Méthylène |
| b₄ | 1535 | 6.67 | Amide II |
| b₅ | 1655 | 6.05 | Amide I |
| b₆ | 1715 | 5.8 | Acides carboxyliques |
| b₇ | 1738 | 5.74 | Esters |

On a également matérialisé deux longueurs d'onde de référence (b₈ et b₉). Ces dernières correspondent à des longueurs d'onde qui sont considérées comme non absorbées de façon significative par les bactéries. Des images formées à ces longueurs d'onde sont représentatives des absorbances du support 15, ainsi que du milieu nutritif présent dans les pores 15₃, ainsi que d'éventuels gaz présents entre le support et le capteur d'image, en particulier le CO₂ ou la vapeur d'eau.

Sur la figure 2A, on observe que dans une bande spectrale s'étendant entre 5.6 µm et 8 µm, l'absorbance de l'alumine est comprise entre 0.98 à 0.2, ce qui correspond à une transmittance comprise entre 0.02 et 0.8.

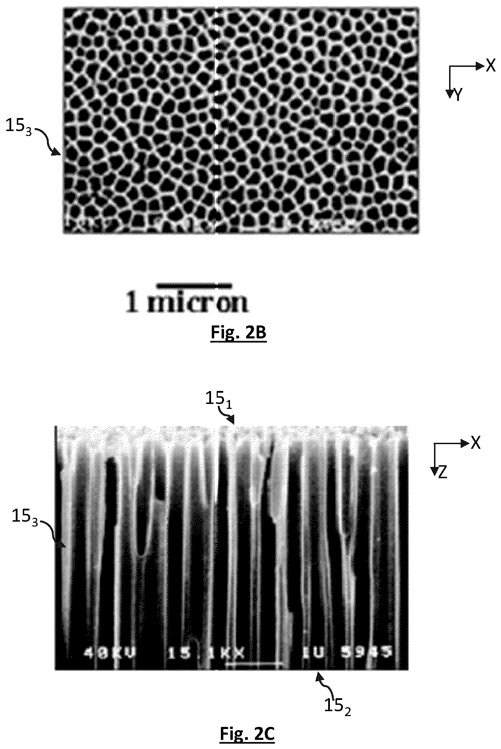

La figure 2B est une image, réalisée au microscope électronique à balayage, d'une face d'un support d'alumine obtenu par oxydation anodique d'une feuille d'aluminium. Un tel matériau est usuellement désigné Porous Anodic Aluminium Oxide (oxyde d'aluminium anodique poreux). On observe un agencement régulier de pores, dont la taille est distribuée autour d'une valeur moyenne de 200 nm. La distribution de la taille des pores est considérée comme homogène. Dans cet exemple, la porosité (fraction volumique des pores), mesurée par traitement d'image, a été estimée à 59%. Plus la porosité augmente, plus la quantité de milieu nutritif, potentiellement absorbant, dans les pores est élevée. Plus la porosité est faible, plus il est difficile au milieu nutritif d'atteindre les microrganismes. D'une façon plus générale, la porosité peut être comprise entre 0.1 % et quelques dizaines de %, par exemple 70% ou 80%.

La figure 2C est une vue en coupe, selon l'épaisseur, du support représenté sur la figure 2B. Dans cet exemple, l'épaisseur du support 15 est de 60 µm. On observe que les pores sont transverses, et s'étendent de la première face 15₁ du support jusqu'à la deuxième face 15₂ du support.

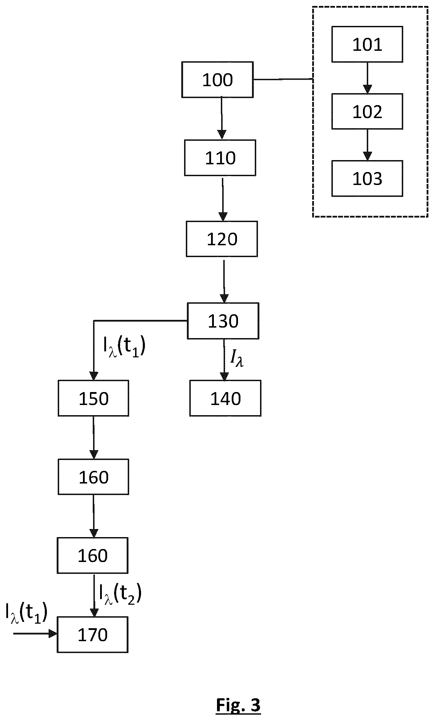

Les inventeurs ont conçu un procédé d'observation de microorganismes, et plus précisément de colonies de microorganismes, dont les principales étapes sont représentées sur la figure 3 et décrites ci-après.

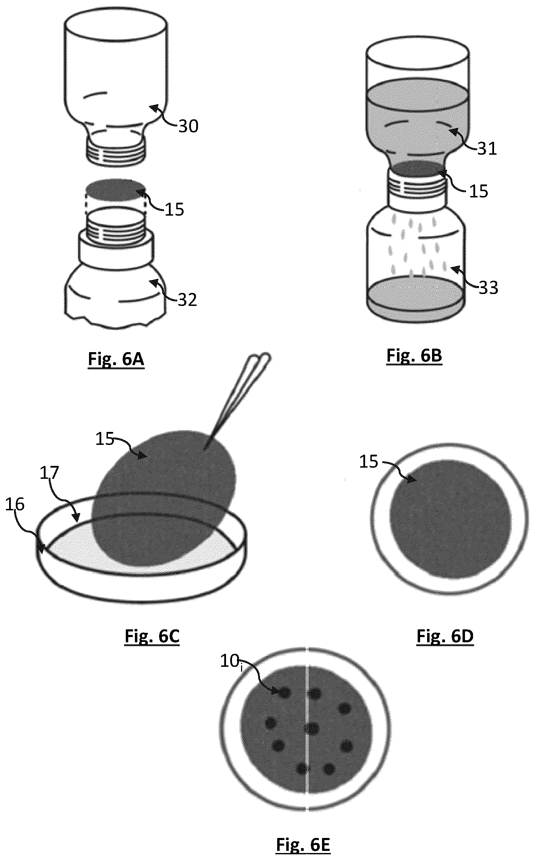

Etape 100 : disposition des microorganismes 10ᵢ sur la première face 15₁ du support poreux 15. Cette étape peut être effectuée par ensemencement par étalement, ou par une variante décrite en lien avec les figures 6A et 6B.

Etape 110 : disposition du support poreux 15 au contact d'un milieu nutritif 17, de telle sorte que les microorganismes puissent se développer. De façon préférée, au cours de cette étape, la deuxième face 15₂ du support poreux 15 est déposée sur la surface libre du milieu nutritif. Au cours de cette étape, le milieu nutritif est contenu dans une enceinte 16, par exemple une boîte de Petri. Comme précédemment décrit, le milieu nutritif 17 diffuse à travers les pores 15₃ pour atteindre les microorganismes 10ᵢ disposés sur la première face 15₁. Cela permet de développement de colonies.

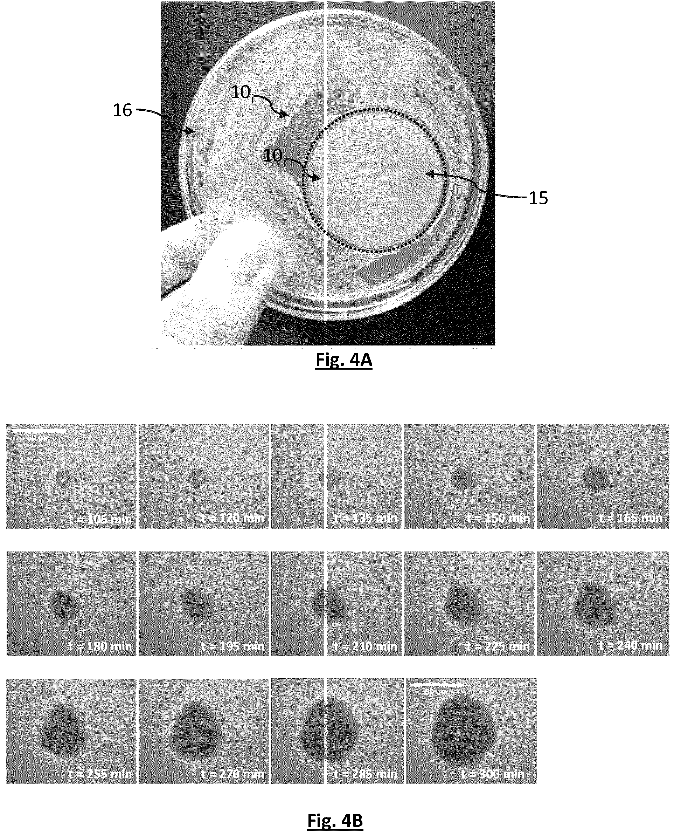

La figure 4A montre une enceinte 16 comportant un milieu nutritif gélosé, sur lequel des bactéries *Enterobacter cloacae* se développent, formant des colonies. On a disposé, sur le milieu nutritif TSA (Tryptic Soy Agar - gélose trypcase soja) un support poreux 15. Sur la figure 4A, le support poreux 15 est contourné par un cadre en pointillés. On observe que les colonies bactériennes se développent de façon comparable sur le support poreux et sur le milieu nutritif en dehors du support poreux. Cela montre la capacité des bactéries à se développer lorsqu'elles dont disposées sur le support poreux 15.

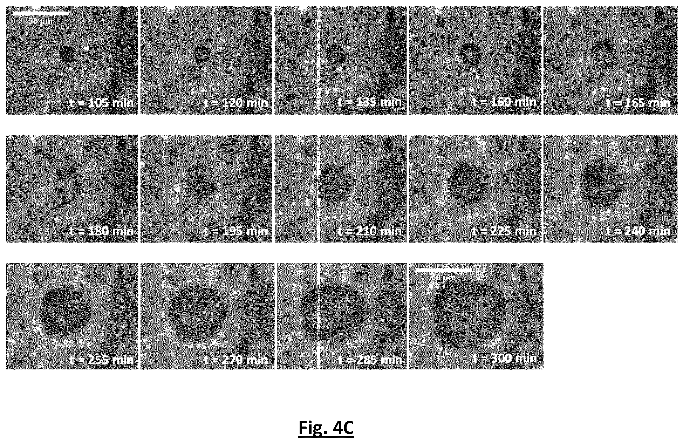

Les figures 4B et 4C sont des images, acquises au cours du temps, de bactéries *Enterobacter cloacae* se développant sur un milieu nutritif gélosé de type LB signifiant Luria Bertani (pouvant également être désigné par le terme bouillon lysogène). Sur la figure 4B, les microorganismes se développent directement à la surface du milieu nutritif. Sur la figure 4C, les microorganismes se développent à la surface d'un support poreux en alumine, dont les pores ont un diamètre de 200 nm. Le support poreux en alumine est disposé sur le milieu nutritif. Ces figures confirment que le développement des microorganismes, sur le support poreux, est comparable au développement directement sur le milieu nutritif. Autrement dit, la présence du support poreux n'affecte pas, ou de façon négligeable, le développement des microorganismes.

Etape 120 : retrait du support poreux 15 du milieu nutritif 17, et déplacement du support poreux relativement à l'enceinte 16. Au cours de cette étape, le support poreux 15 est interposé entre une source de lumière 11 et un capteur d'image 20, comme décrit en lien avec la figure 1C. De préférence, la première face 15₁ est disposée face à la source de lumière 11 et la deuxième face 15₂ est disposée face au capteur d'image 20, mais cela n'est pas nécessaire. On peut prévoir de déposer la première face 15₁ en regard du capteur d'image 20 et la deuxième face 15₂ en regard de la source de lumière 11.

Etape 130 : illumination du support 15 selon au moins une longueur d'onde λ, dans le domaine infra-rouge, comme décrit en lien avec la figure 1C, et acquisition d'une image I_{λ}. L'image acquise permet une observation de colonies présentes sur le support 15. De préférence, l'étape 130 est réitérée selon différentes longueurs d'onde λ, dans le domaine de l'infra-rouge. Lors de chaque illumination, la source de lumière 11 émet une onde lumineuse incidente 12 dont la bande spectrale est préférentiellement étroite, comme préalablement décrit. On obtient ainsi autant d'images I_{λ} que de longueurs d'onde successives d'illumination. Si on met en œuvre N longueurs d'onde λ₁.... λ_{N}, on obtient ainsi N images I_{λ1...} I_{λN}, formant une pile d'images.

### Etape 140 : caractérisation

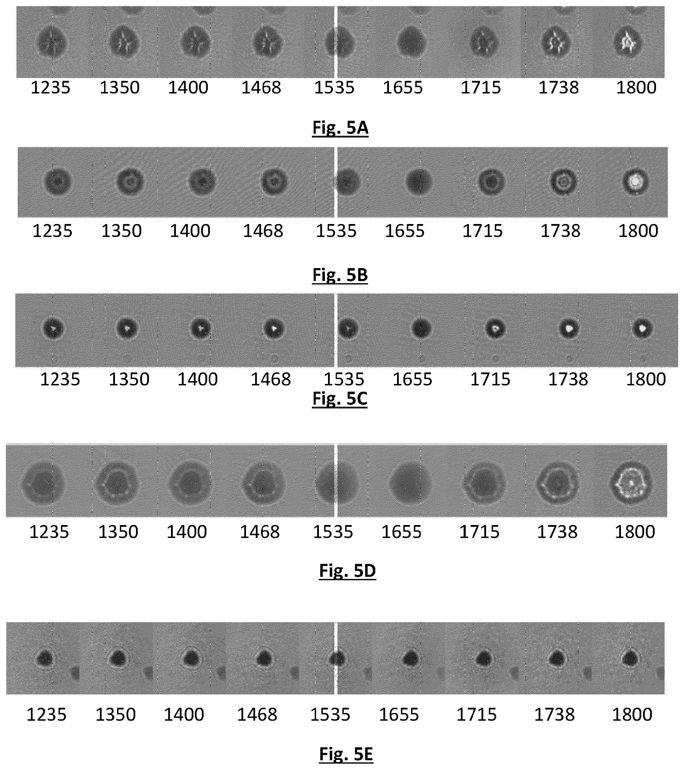

A partir des images de la pile d'image I_{λ1...} I_{λN}, on peut extraire des vignettes I_{i,λ1...} I_{i,λN}, chaque vignette correspondant à une même colonie observée sur chaque image. Chaque vignette est une région d'intérêt d'une image, correspondant à une même colonie. Des exemples d'images, correspondant à respectivement différentes espèces bactériennes, sont décrites en lien avec les figures 5A à 5E.

Chaque vignette I_{i,λ} correspond à une signature de la colonie de microorganismes 10ᵢ à la longueur d'onde I_{i,λ}. La signature dépend :
- de la composition chimique, car l'intensité de chaque vignette I_{i,λ} dépend de l'absorption, par la colonie de microorganismes 10ᵢ examinée, à la longueur d'onde λ.
- de la morphologie de la colonie (morphotype), qui dépend de l'espèce des microorganismes. La morphologie peut être caractérisée par des indicateurs morphologiques, par exemple des moments d'ordre 3 ou 4, usuellement désignés skewness (coefficient d'asymétrie) ou kurtosis (coefficient d'aplatissement). Le morphotype peut également être caractérisé par des moments de Zernike ou de Fourier-Bessel, ces moments étant connus dans le domaine de la classification d'images. Le morphotype peut également être caractérisé par des indicateurs de texture d'image, par exemple une matrice de Haralick.

Les vignettes I_{i,λ} permettent une identification de la bactérie 10ᵢ. La caractérisation des vignettes permet ainsi d'identifier l'espèce bactérienne formant la colonie.

L'étape 140 peut comporter une identification de chaque colonie à partir des vignettes I_{i,λ} correspondant à ladite colonie. Pour cela les images sont traitées par un algorithme de classification, mis en œuvre par l'unité de traitement 21. L'algorithme de traitement peut être par exemple un algorithme d'intelligence artificielle, par exemple un algorithme à apprentissage supervisé. Il peut par exemple s'agir d'un algorithme de type SVM (Support Vector Machine - Machine à vecteurs de support), ou de type réseau de neurones ou des forêts d'arbres décisionnels, usuellement désigné par le terme Random Forest. Le recours à un algorithme à apprentissage supervisé suppose une phase préalable d'apprentissage, en utilisant des images correspondant à des espèces connues de microorganismes.

### Essais expérimentaux

Les étapes 100 à 140 ont été mises en œuvre en utilisant un support de type membrane en oxyde d'aluminium poreux : référence Anodisc (marque déposée) - fabriquant Whatman. L'épaisseur du support était de 60 µm. Le diamètre des pores était de 200 nm.Le support a été stérilisé par autoclave (pression de 1 bar de vapeur d'eau à 121,1°C pendant 15 minutes). Le support a été placé sur un milieu nutritif solide, à 37°C, durant une durée d'incubation de 24h. Après l'incubation, le support a été directement déposé sur un capteur d'image, de type bolomètre de 25 µm de côté - pitch (pas) de 37 µm. La source de lumière était une source laser accordable, référence MIRcat - fabricant Daylight Solutions, permettant d'émettre une onde lumineuse incidente de longueur d'onde comprise entre 5 µm à 11 µm, par pas de 1 cm⁻¹.

Les figures 5A à 5E représentent des vignettes I_{i,λ1...} I_{i,λN}, extraites une pile d'images I_{λ1...} I_{λN} acquises et correspondant respectivement à des bactéries de type *Enterobacter cloacae, Escherichia coli, Candida albicans, Staphyloccocus epidermis, Listeria innocua.* Les vignettes ont été obtenues en illuminant successivement chaque colonie à 10 longueurs d'onde, comprises entre 1235 cm⁻¹ et 1800 cm⁻¹. On a indiqué, en dessous de chaque vignette, le nombre d'onde correspondant à chaque illumination. De telles vignettes ont été utilisées à des fins d'apprentissage d'un algorithme SVM de type SMO (Sequential Minimal Optimization - optimisation minimale séquentielle). Les données d'entrée de l'algorithme étaient des descripteurs de chaque vignette, notamment des moments d'ordre 3 (skewness - coefficient d'aplatissement) ou d'ordre 4 (kurtosis - coefficient d'asymétrie). Les données de sortie étaient la classe d'appartenance correspondant aux données d'entrée. Chaque espèce considérée est associée à une classe.

Les inventeurs ont acquis des vignettes de 1012 colonies. La base de données ainsi constituée a fait l'objet d'une partition en 10 groupes de tailles égales pour une validation croisée. Pour chaque partition, 90% des vignettes (9 groupes) ont été utilisées pour réaliser l'apprentissage supervisé et 10% des vignettes (1 groupe) ont été utilisées à pour tester les performances de l'algorithme de classification. Le tableau 2 représente, dans une matrice de confusion, le pourcentage de classification correctes (entre 0 et 1) résultant des phases de test.

**Tableau 2**

| | *Candida albicans* | *Escherichia coli* | *Enterobacter cloacae* | *Listeria innocua* | *Staphyloccocus epidermis* |
|---|---|---|---|---|---|
| *Candida albicans* | 0.988 | 0.00 | 0.004 | 0.00 | 0.008 |
| *Escherichia coli* | 0.0 | 0.994 | 0.00 | 0.00 | 0.006 |
| *Enterobacter cloacae* | 0.0 | 0.018 | 0.993 | 0.012 | 0.037 |
| *Listeria innocua* | 0.0 | 0.007 | 0.0 | 0.954 | 0.039 |
| *Staphyloccocus epidermis* | 0.004 | 0.019 | 0.011 | 0.026 | 0.94 |

Le tableau 2 montre que les différentes espèces de microorganismes peuvent être identifiées avec un niveau de confiance satisfaisant. Cela atteste de la pertinence de l'invention.

### Variantes

Selon une première variante, le support poreux 15 est également utilisé en tant que filtre, de façon à retenir des microorganismes dans un milieu fluide, préalablement à leur identification. Cette variante est illustrée sur les figures 6A à 6F. Cela correspond à des sous-étapes 101 à 103 de l'étape 100 précédemment décrite.

Sous-étape 101 : montage du support. Sur la figure 6A, on a représenté un réservoir amont 30 et un réservoir aval 32. Le support poreux 15 est disposé entre le réservoir amont 30 et le réservoir aval 32. Le réservoir amont est destiné à recevoir un fluide 31 à analyser, susceptible de comporter des microorganismes. Dans cet exemple, le fluide est un liquide.

Sous-étape 102 : filtration. Cette étape est représentée sur la figure 6B. Le réservoir amont 30 est rempli du fluide 31 à analyser. Ce dernier s'écoule à travers le support poreux 15. Les éventuels microorganismes 10ᵢ présents dans le fluide sont retenus par le filtre. Cette étape permet de concentrer, sur le support poreux 15, les microorganismes initialement présents dans le réservoir amont 30, ce dernier pouvant avoir un volume important, par exemple 100 mL. Le fluide filtré 33 peut être récupéré dans le réservoir aval 32.

Sous-étape 103 : retrait du filtre : le support poreux 15 est retiré de façon à être placé à la surface d'un milieu nutritif 17.

Suite à cela, on dépose le support poreux 15 à la surface du milieu nutritif 17, le milieu nutritif occupant une enceinte 16. Cf. figure 6C. Les figures 6D et 6E schématisent le développement de colonies de microorganismes sur le support poreux au contact du milieu nutritif, ce qui correspond à l'étape 110 préalablement décrite. Les étapes 120 à 140 peuvent ensuite être mises en œuvre, de façon à identifier les espèces de microorganismes et/ou à compter le nombre de colonies formées, et cela éventuellement pour chaque espèce identifiée. Le nombre de colonies peut être ramené au volume (ou à la masse) de fluide 31, ce qui permet de calculer une charge microbienne par unité de masse ou de volume : unité formant colonie (ufc) par gramme ou par millilitre par exemple)

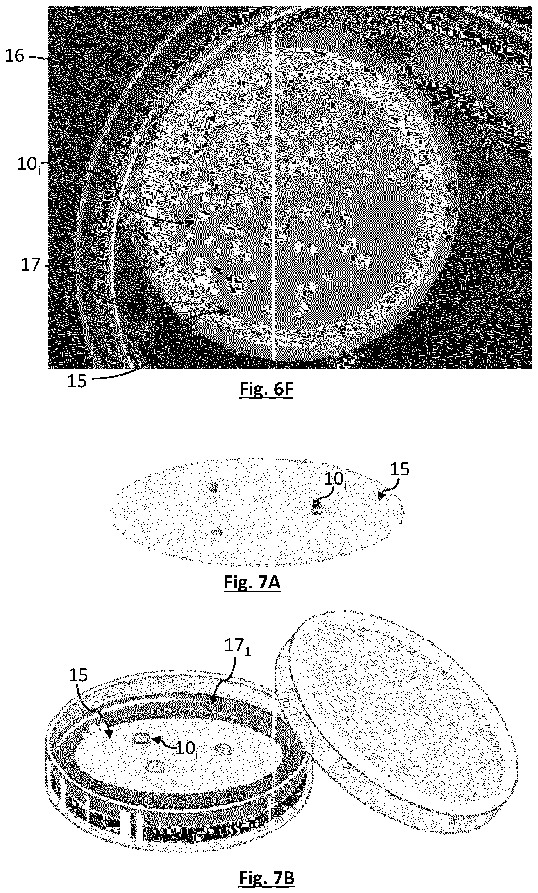

Les inventeurs ont mis en œuvre les étapes schématisées sur les figures 6A à 6E en disposant un support poreux d'alumine, dont la taille moyenne des pores était de 200 nm. Le support poreux a été placé sur un milieu nutritif chromogène. Un tel milieu a la propriété de colorer les microorganismes se développant. La figure 6F est une photographie d'un support poreux mis en contact avec un milieu nutritif chromogène durant une durée d'incubation de 24 heures. Le milieu nutritif chromogène était une gélose chromogène de type Chromid (marque déposée) CPS (marque déposée) - fabriquant Biomérieux. On observe la présence de taches sombres, qui correspondent à des colonies de bactéries *(Escherichia coli*) colorées suite à leur développement dans le milieu nutritif chromogène.

Un exemple d'application de la première variante est l'analyse bactériologique de l'eau destinée à la consommation humaine. De façon alternative, le fluide 31 est un gaz. L'invention peut permettre de retenir et d'analyser des microorganismes portés dans un gaz, par exemple de l'air : air industriel, air en milieu hospitalier.

Selon une deuxième variante, la caractérisation des microorganismes consiste non pas à les identifier, mais à évaluer leur capacité à se développer. Cela permet d'obtenir des indicateurs de sensibilité à l'égard d'un agent bactéricide ou d'un agent bactériostatique par exemple un antibiotique. Un exemple d'un indicateur est la concentration minimale inhibitrice (CMI), qui correspond à une concentration minimale d'antibiotique à laquelle le développement de bactéries est inhibé.

Ainsi, le milieu nutritif peut comporter un antibiotique selon une concentration connue, ou selon un gradient spatial de concentration connu, par exemple concentration importante au centre et concentration faible à la périphérie. Des images du support 15 peuvent être réalisées respectivement à un instant initial t₁ et à un deuxième instant t₂. Entre les instants t₁ et t₂, le support est disposé à la surface d'un milieu nutritif comportant un antibiotique. La comparaison des images obtenues respectivement à l'instant initial t₁ et au deuxième instant t₂ permet d'évaluer le développement des colonies entre les deux instants. On peut alors caractériser la capacité du microorganisme analysé à se développer en présence de l'antibiotique.

Selon une possibilité, entre les instants t₁ et t₂, le milieu nutritif comporte, outre un antibiotique, un marqueur isotopique présent selon une concentration enrichie par rapport à la concentration naturelle. Le marqueur isotopique est une molécule comportant un isotope stable d'un élément. Il peut par exemple s'agir de Deutérium (D), substitué à de l'Hydrogène (H). Un tel marqueur obtenu en utilisant de l'eau lourde (D₂O). Le milieu nutritif marqué peut être par exemple un milieu de Mueller-Hinton comportant de l'eau lourde, ainsi qu'une concentration connue (ou un gradient spatial de concentration connu). Au deuxième instant, le support poreux 15 est illuminé selon une longueur d'onde absorbée par une liaison chimique impliquant l'isotope. Il peut par exemple s'agir de la liaison Carbone - Deutérium lorsque l'isotope est le Deutérium. La mise en évidence, par une image, de la présence d'une telle liaison permet de conclure au métabolisme actif dans le milieu nutritif marqué. Inversement, l'absence de mise en évidence d'une telle liaison conduit à conclure à l'absence de métabolisme actif de la bactérie.

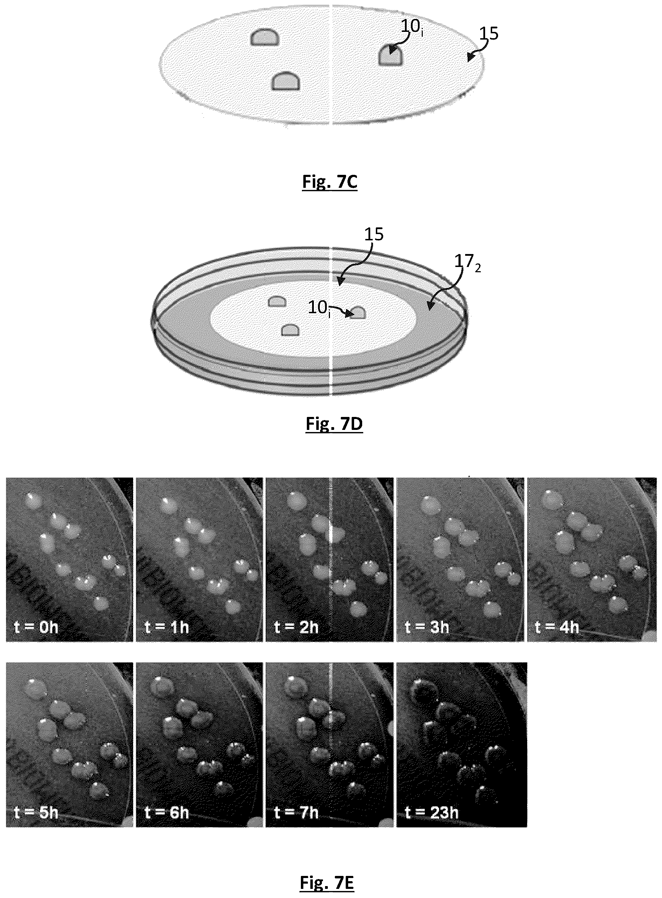

Cette variante correspond aux étapes 100 à 130 et 150 à 170 représentées sur la figure 3, ainsi qu'aux figures 7A à 7E. Les étapes 100 à 130 sont mises en œuvre comme préalablement décrit. Les figures 7A et 7B illustrent les étapes 100 (ensemencement) et 110 (incubation). Lors de l'étape 110, le milieu nutritif utilisé est un milieu initial 17₁, ne comportant pas d'antibiotique. Lors de l'étape 120, on retire le support poreux 15 du milieu nutritif initial, cf figure 7C. L'étape 130 est mise en œuvre à un instant initial t₁, en utilisant un dispositif tel que représenté sur la figure 1C. Cela conduit à l'obtention d'images initiales I_{λ}(t₁).

Au cours de l'étape 150, le support poreux est disposé au contact d'un deuxième milieu nutritif 17₂. Cf. figure 7D. Le deuxième milieu nutritif 17₂ comporte une concentration d'antibiotique connue, ou un gradient spatial de concentration d'antibiotique connu. Le deuxième milieu 17₂ peut comporter un marqueur isotopique, par exemple sous l'effet de l'ajout d'eau lourde.

Au cours de l'étape 160, le support poreux est retiré du deuxième milieu nutritif 17₂, puis disposé dans un dispositif d'observation tel que représenté sur la figure 1C.

Au cours de l'étape 170, on forme des deuxièmes images I_{λ}(t₂), à un deuxième instant t₂, postérieur à l'instant initial t₁. La comparaison des images initiales I_{λ}(t₁), et des deuxièmes images I_{λ}(t₂) permet d'évaluer la capacité des microorganismes à métaboliser dans le deuxième milieu.

Le recours à un marqueur isotopique peut être remplacé par un marqueur comportant une liaison covalente connue, présentant une absorbance exploitable à une longueur d'onde d'illumination de la source de lumière. Il peut s'agir par exemple de la liaison C ≡ N, présentant une longueur d'onde d'absorption à 2200 cm⁻¹. La formation d'images à ladite longueur d'onde permet d'évaluer l'activité métabolique du microorganisme dans le deuxième milieu.

Le recours à un marqueur isotopique peut être remplacé en utilisant un milieu chromogène, apte à induire une modification de la couleur des microorganismes sous l'effet de leur métabolisme. La figure 7E illustre une telle alternative. Cette figure représente une évolution de la couleur de colonies d'*Escherichia coli.* Ces colonies ont tout d'abord fait l'objet d'une mise en culture sur un support poreux reposant sur un milieu nutritif initial (TSA Tryptic Soy Agar - gélose trypcase soja). Le support poreux a ensuite été déposé sur un milieu chromogène de type Chromid (marque déposée) CPS (marque déposée) - fabriquant Biomérieux. On observe une coloration progressive des bactéries, de la couleur blanche à la couleur rouge.

L'invention permet une caractérisation simultanée d'un grand nombre de microorganismes, tout en n'étant pas destructif. Elle ne nécessite pas d'instrumentation complexe et est particulièrement simple à mettre en œuvre. En outre, elle est aisément automatisable.

## Revendications

1. Procédé de caractérisation de microorganismes, comportant :
a) dépôt de microorganismes (10ᵢ) sur un support poreux (15), le support poreux comportant une première face (15₁) et une deuxième face (15₂), et des pores (15₃) s'étendant de la première face jusqu'à la deuxième face, les microorganismes étant retenus sur la première face ;
b) disposition du support poreux à la surface d'un milieu nutritif (17, 17₂), contenu dans une enceinte (16), le support poreux étant disposé de telle sorte que la deuxième face est disposée au contact du milieu nutritif, de façon que le milieu nutritif diffuse de la deuxième face vers la première face, à travers les pores ;
c) déplacement du support poreux par rapport à l'enceinte ;
d) positionnement du support poreux entre une source de lumière infra-rouge (11) et un capteur d'image (20), la source de lumière étant configurée pour émettre une onde lumineuse incidente dans une longueur d'onde d'émission (λ), le support poreux (15) transmettant tout ou partie de l'onde lumineuse incidente à la longueur d'onde d"émission (λ) ;
e) illumination des microorganismes, disposés sur le support poreux, par la source de lumière et acquisition d'une image (I_{λ}, I_{λ}(t₂)) par le capteur d'image, à la longueur d'onde d'émission, l'image permettant une observation d'au moins une colonie de microorganismes ;
f) caractérisation de la colonie de microorganismes à partir de l'image acquise lors de l'étape e) ;
le procédé étant **caractérisé en ce que** :
- l'étape e) est répétée en illuminant les microorganismes successivement à différentes longueurs d'onde d'émission (λ₁....λ_{N}), de façon à obtenir autant d'images (I_{λ1...} I_{λN}) que de longueurs d'onde d'émission ;
- lors de l'étape f), la caractérisation est effectuée à partir des images (I_{λ1...} I_{λN}) acquises lors de l'étape e),
la caractérisation étant :
- une identification de l'espèce des microorganismes formant la colonie ;
- ou une détermination de la capacité des microorganismes à se développer dans le milieu nutritif.

2. Procédé selon l'une quelconque des revendications précédentes, dans lequel le support poreux transmet au moins 1% de la lumière dans la longueur d'onde d'émission (λ), ou dans chaque longueur d'onde d'émission (λ₁....λ_{N}).

3. Procédé selon l'une quelconque des revendications précédentes, dans lequel l'étape f) comporte une extraction d'une vignette (I_{i,λ}) à partir de chaque image (I_{λ}) acquise lors de l'étape e), la vignette étant une région d'intérêt de l'image acquise correspondant à la colonie de microorganismes (10ᵢ).

4. Procédé selon la revendication 3, dans lequel lors de l'étape f), chaque vignette (I_{i,λ1...} I_{i,λN}) forme une donnée d'entrée d'un algorithme à apprentissage supervisé, de façon à caractériser les microorganismes formant une colonie.

5. Procédé selon l'une quelconque des revendications précédentes, dans lequel l'étape a) comporte un ensemencement du support poreux.

6. Procédé selon l'une quelconque des revendications 1 à 4, dans lequel l'étape a) comporte un écoulement d'un fluide (31), susceptible de comporter des microorganismes, à travers le support poreux, de la première face vers la deuxième face, le support agissant en tant que filtre, de façon à retenir les microorganismes sur la première face, tout en autorisant un écoulement du fluide à travers les pores.

7. Procédé selon l'une quelconque des revendications précédentes, dans lequel lors de l'étape b), lequel le milieu nutritif :
- comporte des molécules marquées par un isotope ;
- ou est un milieu chromogène ;
- ou comporte des molécules présentant une liaison chimique absorbant la lumière à la longueur d'onde d'émission ;
de telle sorte que l'image formée lors de l'étape e), ou chaque image formée lors de l'étape e) est représentative d'une activité métabolique des microorganismes au contact avec le milieu nutritif.

8. Procédé selon l'une quelconque des revendications précédentes, dans lequel lors de l'étape b), le milieu nutritif comporte un agent bactéricide ou un agent bactériostatique, par exemple un antibiotique, de telle sorte que l'image formée lors de l'étape e), ou chaque image formée lors de l'étape e) est représentative d'une activité métabolique des microorganismes lorsqu'ils sont en contact avec le milieu nutritif.

9. Procédé selon la revendication 7 ou la revendication 8, comportant, suite à l'étape a) et préalablement à l'étape b), une acquisition d'une image initiale du support poreux, de telle sorte que l'activité métabolique est déterminée en fonction d'une comparaison entre l'image initiale (I_{λ}(t₁)) et au moins une image (I_{λ}(t₂)) acquise lors de l'étape e.

10. Procédé selon l'une quelconque des revendications précédentes, dans lequel l'épaisseur du support poreux, entre la première face et la deuxième face, est inférieure à 1 mm ou à 500 µm.

11. Procédé selon l'une quelconque des revendications précédentes, dans lequel le diamètre ou la plus grande diagonale de chaque pore est compris entre 5 nm et 5 µm, et de préférence ente 20 nm et 500 nm.

12. Procédé selon l'une quelconque des revendications précédentes, dans lequel le support poreux est obtenu à partir d'un matériau choisi parmi : l'alumine, le silicium, le germanium, le sulfure de zinc, le nitrure de silicium, le séléniure de zinc, un verre de chalcogénure, le fluorure de calcium, le bromure de potassium.

13. Procédé selon l'une quelconque des revendications précédentes, dans lequel l'étape a) comporte un écoulement d'un fluide à analyser à travers le support poreux, de façon que des microorganismes présents dans le fluide sont retenus par le support poreux, le support poreux agissant comme un filtre.

14. Procédé selon l'une quelconques des revendications précédentes, comportant :
- une première mise en œuvre des étapes b) à e), le support étant disposé, lors de l'étape b) sur un milieu de culture initial ;
- une deuxième mise en œuvre des étapes b) à e), le support étant disposé, lors de la deuxième étape b), sur un deuxième milieu de culture, différent ou identique du milieu de culture initial.

## Patentansprüche

1. Verfahren zur Charakterisierung von Mikroorganismen, umfassend:
a) Aufbringen von Mikroorganismen (10ᵢ ) auf einen porösen Träger (15), wobei der poröse Träger eine erste Seite (15₁ ) und eine zweite Seite (15₂ ) sowie Poren (15₃ ) aufweist, die sich von der ersten Seite bis zur zweiten Seite erstrecken, wobei die Mikroorganismen auf der ersten Seite zurückgehalten werden;
b) Anordnen des porösen Trägers auf der Oberfläche eines Nährmediums (17, 17₂ ), das in einem Gehäuse (16) enthalten ist, wobei der poröse Träger so angeordnet ist, dass die zweite Seite in Kontakt mit dem Nährmedium steht, so dass das Nährmedium von der zweiten Seite zur ersten Seite durch die Poren diffundiert;
c) Bewegung des porösen Trägers relativ zum Gehäuse;
d) Positionierung des porösen Trägers zwischen einer Infrarotlichtquelle (11) und einem Bildsensor (20), wobei die Lichtquelle so konfiguriert ist, dass sie eine einfallende Lichtwelle mit einer Emissionswellenlänge (λ) aussendet, wobei der poröse Träger (15) die gesamte oder einen Teil der einfallenden Lichtwelle mit der Emissionswellenlänge (λ) durchzulassen;
e) Beleuchtung der auf dem porösen Träger angeordneten Mikroorganismen durch die Lichtquelle und Erfassung eines Bildes (I_{λ}, I_{λ}(t₂)) durch den Bildsensor bei der Emissionswellenlänge, wobei das Bild die Beobachtung mindestens einer Kolonie von Mikroorganismen ermöglicht;
f) Charakterisierung der Kolonie von Mikroorganismen anhand des in Schritt e) aufgenommenen Bildes;
wobei das Verfahren **dadurch gekennzeichnet ist, dass** :
- Schritt e) wiederholt wird, indem die Mikroorganismen nacheinander mit verschiedenen Emissionswellenlängen (λ₁ ....λ_{N} ) beleuchtet werden, so dass ebenso viele Bilder (I_{λ1...} I_{λN} ) wie Emissionswellenlängen erhalten werden;
- in Schritt f) die Charakterisierung anhand der in Schritt e) erfassten Bilder (I_{λ1...} I_{λN}) durchgeführt wird;
wobei die Charakterisierung Folgendes umfasst:
- eine Identifizierung der Art der Mikroorganismen, die die Kolonie bilden;
- oder eine Bestimmung der Fähigkeit der Mikroorganismen, sich in dem Nährmedium zu entwickeln.

2. Verfahren nach einem der vorstehenden Ansprüche, wobei der poröse Träger mindestens 1 % des Lichts in der Emissionswellenlänge ( 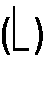 ) oder in jeder Emissionswellenlänge (λ₁ ....λ_{N} ) durchlässt.

3. Verfahren nach einem der vorstehenden Ansprüche, wobei Schritt f) das Extrahieren eines Bildausschnitts (I_{i,λ}) aus jedem in Schritt e) erfassten Bild (I_{λ}) umfasst, wobei der Bildausschnitt ein interessierender Bereich des erfassten Bildes ist, der der Kolonie von Mikroorganismen (10ᵢ ) entspricht.

4. Verfahren nach Anspruch 3, wobei in Schritt f) jede Miniaturansicht (I_{i,λ1...} I_{i,λN} ) Eingabedaten für einen überwachten Lernalgorithmus bildet, um die Mikroorganismen zu charakterisieren, die eine Kolonie bilden.

5. Verfahren nach einem der vorstehenden Ansprüche, wobei Schritt a) eine Besiedlung des porösen Trägers umfasst.

6. Verfahren nach einem der Ansprüche 1 bis 4, wobei der Schritt a) ein Fließen einer Flüssigkeit (31), die Mikroorganismen enthalten kann, durch den porösen Träger von der ersten Seite zur zweiten Seite umfasst, wobei das Medium als Filter wirkt, um die Mikroorganismen auf der ersten Seite zurückzuhalten, während es gleichzeitig ein Fließen der Flüssigkeit durch die Poren ermöglicht.

7. Verfahren nach einem der vorstehenden Ansprüche, wobei in Schritt b) das Nährmedium:
- mit einem Isotop markierte Moleküle enthält;
- oder ein chromogenes Medium ist;
- oder Moleküle mit einer chemischen Bindung aufweist, die Licht mit der Emissionswellenlänge absorbiert;
sodass das in Schritt e) erzeugte Bild oder jedes in Schritt e) erzeugte Bild repräsentativ für eine Stoffwechselaktivität der Mikroorganismen in Kontakt mit dem Nährmedium ist.

8. Verfahren nach einem der vorstehenden Ansprüche, wobei in Schritt b) das Nährmedium ein bakterizides oder bakteriostatisches Mittel, beispielsweise ein Antibiotikum, enthält, so dass das in Schritt e) erzeugte Bild oder jedes in Schritt e) erzeugte Bild die Stoffwechselaktivität der Mikroorganismen im Kontakt mit dem Nährmedium darstellt.

9. Verfahren nach Anspruch 7 oder Anspruch 8, das nach Schritt a) und vor Schritt b) eine Erfassung eines Ausgabebildes des porösen Trägers umfasst, so dass die Stoffwechselaktivität anhand eines Vergleichs zwischen dem Ausgabebild (I_{λ} (t₁ )) und mindestens einem in Schritt e aufgenommenen Bild (I_{λ} (t₂ )) bestimmt wird.

10. Verfahren nach einem der vorstehenden Ansprüche, wobei die Dicke des porösen Trägers zwischen der ersten Seite und der zweiten Seite weniger als 1 mm oder 500 µm beträgt.

11. Verfahren nach einem der vorstehenden Ansprüche, wobei der Durchmesser oder die größte Diagonale jeder Pore zwischen 5 nm und 5 µm und vorzugsweise zwischen 20 nm und 500 nm liegt.

12. Verfahren nach einem der vorstehenden Ansprüche, wobei der poröse Träger aus einem Material hergestellt ist, das aus Aluminiumoxid, Silizium, Germsanium, Zinksulfid, Siliziumnitrid, Zinkselenid, einem Chalkogenidglas, Calciumfluorid oder Kaliumbromid ausgewählt ist.

13. Verfahren nach einem der vorstehenden Ansprüche, wobei Schritt a) das Durchfließen einer zu analysierenden Flüssigkeit durch den porösen Träger umfasst, so dass in der Flüssigkeit vorhandene Mikroorganismen durch den porösen Träger zurückgehalten werden, wobei der poröse Träger als Filter wirkt.

14. Verfahren nach einem der vorstehenden Ansprüche, umfassend:
- eine erste Durchführung der Schritte b) bis e), wobei der Träger in Schritt b) auf einem anfänglichen Nährmedium angeordnet ist;
- eine zweite Durchführung der Schritte b) bis e), wobei der Träger in Schritt b) auf einem zweiten Nährmedium angeordnet ist, das sich vom anfänglichen Nährmedium unterscheidet oder mit diesem identisch ist.

## Claims

1. A method for characterizing microorganisms, comprising:
a) depositing microorganisms (10ᵢ) on a porous carrier (15), the porous carrier comprising a first face (15₁) and a second face (15₂), and pores (15₃) extending from the first face to the second face, the microorganisms being retained on the first face;
b) placing the porous carrier on the surface of a nutrient medium (17, 17₂) contained in a chamber (16), the porous carrier being placed such that the second face is placed in contact with the nutrient medium, so that the nutrient medium diffuses from the second face to the first face, through the pores;
c) moving the porous carrier with respect to the chamber;
d) positioning the porous carrier between an infrared light source (11) and an image sensor (20), the light source being configured to emit an incident light wave at an emission wavelength (λ), the porous carrier (15) transmitting all or some of the incident light wave at the emission wavelength (λ);
e) illuminating the microorganisms, placed on the porous carrier, with the light source and acquiring an image (I_{λ}, I_{λ}(t₂)) with the image sensor, at the emission wavelength, the image allowing at least one colony of microorganisms to be observed;
f) characterizing the colony of microorganisms on the basis of the image acquired in step e);
wherein the method is **characterized in that**:
- step e) is repeated, the microorganisms being successively illuminated at various emission wavelengths (λ₁....λ_{N}), so as to obtain as many images (I_{λ1 ...} I_{λN}) as there are emission wavelengths;
- in step f), the characterization is performed on the basis of the images (I_{λ1 ...} I_{λN}) acquired in step e) ;
wherein the characterization is:
- identifying the species of the microorganisms forming the colony;
- or determining the ability of the microorganisms to develop in the nutrient medium.

2. The method as claimed in any one of the preceding claims, wherein the porous carrier transmits a least 1% of the light at the emission wavelength (λ), or at each emission wavelength (λ₁....λ_{N}).

3. The method as claimed in any one of the preceding claims, wherein step f) comprises extracting a thumbnail (I_{i,λ}) from each image (I_{λ}) acquired in step e), the thumbnail being a region of interest of the acquired image corresponding to the colony of microorganisms (10i).

4. The method as claimed in claim 3, wherein, in step f), each thumbnail (I_{i,λ1 ...} I_{i,λN}) forms an input datum of a supervised-learning algorithm, with a view to characterizing the microorganisms forming a colony.

5. The method as claimed in any one of the preceding claims, wherein step a) comprises seeding the porous carrier.

6. The method as claimed in any one of claims 1 to 4, wherein step a) comprises making a fluid (31), liable to contain microorganisms, flow through the porous carrier, from the first face to the second face, the carrier acting as a filter, so as to retain microorganisms on the first face, while permitting the fluid to flow through the pores.

7. The method as claimed in any one of the preceding claims, wherein, in step b), the nutrient medium:
- contains molecules labelled with an isotope;
- or is a chromogenic substrate;
- or comprises molecules having a chemical bond that absorbs light at the emission wavelength;
such that the image formed in step e), or each image formed in step e), is representative of a metabolic activity of the microorganisms in contact with the nutrient medium.

8. The method as claimed in any one of the preceding claims, wherein, in step b), the nutrient medium comprises a bactericide or a bacteriostat, an antibiotic for example, such that the image formed in step e), or each image formed in step e), is representative of a metabolic activity of the microorganisms when they are in contact with the nutrient medium.

9. The method as claimed in claim 7 or claim 8, comprising, following step a) and prior to step b), acquiring an initial image of the porous carrier, such that metabolic activity may be determined via a comparison between the initial image (I_{λ}(t₁)) and at least one image (I_{λ}(t₂)) acquired in step e).

10. The method as claimed in any one of the preceding claims, wherein the thickness of the porous carrier, between the first face and the second face, is smaller than 1 mm or than 500 µm.

11. The method as claimed in any one of the preceding claims, wherein the diameter or largest diagonal of each pore is comprised between 5 nm and 5 µm, and preferably between 20 nm and 500 nm.

12. The method as claimed in any one of the preceding claims, wherein the porous carrier is obtained from a material chosen from: alumina, silicon, germanium, zinc sulfide, silicon nitride, zinc selenide, a chalcogenide glass, calcium fluoride, and potassium bromide.

13. The method as claimed in any one of the preceding claims, wherein step a) comprises flowing a fluid to be analysed through the porous carrier, such that microorganisms within the fluid are retained by the porous medium, the porous carrier acting as a filter.

14. The method as claimed in any one of the preceding claims, comprising:
- a first implementation of steps b) to e), the carrier being placed, during step b), on an initial nutrient medium;
- a second implementation of steps b) to e), the carrier being placed, during the second step b), on a second nutrient medium, which may be different from or identical to the initial nutrient medium.
